# EUROPÄISCHE PATENTSCHRIFT

(11) **EP 4 474 648 B1**
(45) Veröffentlichungstag und Bekanntmachung des Hinweises auf die Patenterteilung: **09.09.2026**
(21) Anmeldenummer: 24179558.2
(22) Anmeldetag: 03.06.2024
(51) Int. Cl.: F04B 43/12, A61M 1/14, A61M 60/279, A61M 60/113

(54) **PERISTALTIKPUMPE**
PERISTALTIC PUMP
POMPE PÉRISTALTIQUE

(30) Priorität: 07.06.2023 DE 102023115055
(43) Veröffentlichungstag der Anmeldung: 11.12.2024
(73) Patentinhaber: B. Braun Avitum AG, 34212 Melsungen (DE)
(72) Erfinder: JAKOBI, Marco, 37318 Uder (DE)
(74) Vertreter: Winter, Brandl - Partnerschaft mbB

(56) Entgegenhaltungen:
- EP-B1- 1 749 549
- DE-A1- 102019 133 969

## Beschreibung

Die Erfindung betrifft ein eine Schlauchrollen- bzw. Peristaltikpumpe, d.h. eine Verdrängerpumpe, bei der das zu fördernde Medium durch äußere mechanische Verformung eines Schlauches durch diesen hindurchgedrückt wird, gemäß dem Oberbegriff des Patentanspruchs 1. Derartige Pumpen werden häufig zum Fördern von Fluid, insbesondere Blut, in einer Vorrichtung zur extrakorporalen Blutbehandlung, insbesondere in einer Dialysemaschine, verwendet. Das Fluid wird dabei mittels der Peristaltikpumpe von einer Niederdruckseite zu einer Hochdruckseite gefördert, wobei eine elastisch verformbare und zwischen der Niederdruckseite und der Hochdruckseite angeordnete Fluidleitung in Form eines Schlauchsegments, welches als Pumpensegment bezeichnet wird, zwischen einer Stützfläche eines Pumpenbetts und einem gegenüber dieser rotierenden Rotor mit zumindest zwei Quetschelementen verformt, insbesondere zusammengequetscht.

Eine solche Schlauchrollenpumpe ist beispielsweise aus dem Dokument DE 10 2019 133 969 A1 bekannt.

Bei einer anderen bekannten Peristaltikpumpe, wie sie beispielsweise aus dem Dokument EP 1 749 549 B1 bekannt und ist, sind - wie in Figur 8 bis 11 schematisch dargestellt - am Rotor 310 zwei diametral zueinander versetzt angeordnete Quetschelemente 320 in Form von gefedert gelagerten Andruckrollen vorgesehen, und die Stützfläche 330 wird von einer Kreissegmentfläche gebildet, die sich über einen ausreichend großen Zentriwinkel WZS größer als 180° erstreckt.

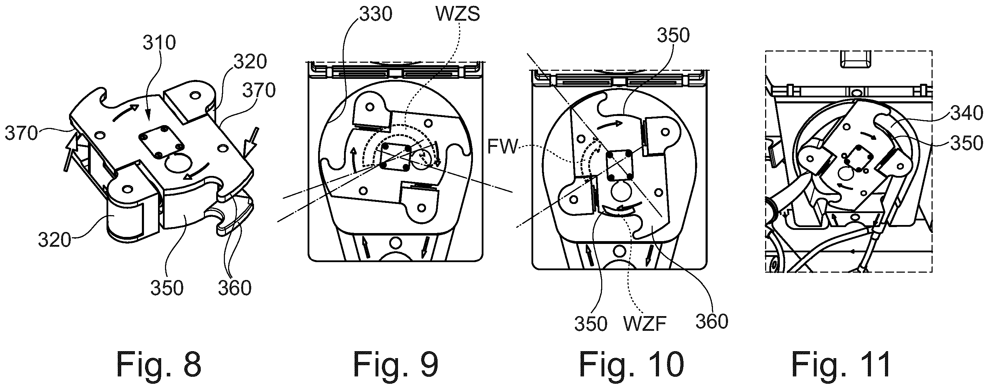

Zur zusätzlichen Fixierung des in die Peristaltikpumpe, d.h. in das Pumpenbett eingelegten Schlauchsegments 340 (siehe Figur 11), befindet sich in der Mitte zwischen den beiden Andruckrollen 320 jeweils eine kreissegmentartig ausgeführte Führungsfläche 350, die sich über einen Zentriwinkel WZF (siehe Figur 10) von etwa 30° erstreckt. Der Führungsfläche 350 vorlaufend trägt der Rotor 310 ein Paar von Führungsvorsprüngen 360, die plattenartig ausgebildet sind und sich radial bis in die Nähe der Stützfläche 330 erstrecken, so dass das Schlauchsegments 340 zwischen den Führungsvorsprüngen 360 axial gefangen ist. Am vorlaufenden Ende der Führungsvorsprünge 360 ist eine Kante 370 ausgebildet, die beim manuellen Ausfädeln des Schlauchsegments 340 wie folgt genutzt wird:

Wenn das Schlauchsegment 340 - wie in Figur 11 gezeigt - aus dem Gehäuse der Peristaltikpumpe ausgefädelt werden soll, besteht bei der bekannten Peristaltikpumpe die Gefahr, dass das Schlauchsegment 340 dann, wenn der Rotor 310 eine ungünstige Drehlage einnimmt, eingeklemmt wird. Figur 11 zeigt einen solchen Zustand. Die an den Führungsvorsprüngen 360 ausgebildeten Kanten 370 können nur dann sinnvoll zum Ausfädeln des Schlauchsegment 340 genutzt werden, wenn sich der Rotor 310 in Drehlagen befindet, die zwischen den Positionen gemäß Figur 9 und Figur 10 liegen. Mit anderen Worten, der die Peristaltikpumpe bedienenden Person steht zum Ausfädeln des Schlauchsegments nur ein begrenztes Winkelfenster FW (siehe Figur 10) von unter 90° zur Verfügung, in dem die Wahrscheinlichkeit des Einklemmens des Schlauchsegments 340 geringer ist.

Der Erfindung liegt daher die Aufgabe zugrunde, die gattungsgemäße Peristaltikpumpe derart weiterzubilden, dass das Ausfädeln des in der Peristaltikpumpe aufgenommenen Schlauchsegments vereinfacht ist, wobei das Schlauchsegment weitestgehend vor Beschädigungen geschützt sein soll.

Diese Aufgabe wird durch die Merkmale des Anspruchs 1 gelöst.

Die vorgeschlagene Peristaltikpumpe mit den Merkmalen des Oberbegriffs des Anspruchs 1 zeichnet sich durch eine dem jeweiligen Quetschelement entgegen der Drehrichtung des Rotors benachbart angeordnete, sich zur Stützfläche hin erstreckende Abgleitkante aus, die an den Rotor drehfest angeschlossen und vom eingelegten Schlauchsegment untergreifbar ist. Durch die neuerungsgemäße Anordnung der Abgleitkante bewirkt die Zugbewegung am Schlauchsegments bei der manuellen Entnahme bzw. beim Ausfädeln des Schlauchsegments schon bei sehr kleinen Zugkräften ein Drehen des Rotors, wodurch ein Einklemmen des Schlauches zwischen Quetschelement und Pumpenbett zuverlässig verhindert wird. Die auf das Schlauchsegment ausgeübten Zug- und Reibungskräfte werden somit verringert, wodurch es gelingt, das Schlauchsegment beim Ausfädelvorgang sehr schonend zu behandeln. Dabei ergibt sich der zusätzliche Vorteil, dass aufgrund der Nähe der Abgleitkante zu den Quetschelementen bzw. Andruckrollen der Bereich, in dem sich der Rotor beim manuellen Ausfädeln befinden muss, um das Einklemmen des Schlauches zu vermeiden, größer wird, so dass das Ausfädeln des Schlauchsegments insgesamt vereinfacht wird.

Die Abgleitkante kann an unterschiedlichsten Komponenten der Peristaltikpumpe angeordnet sein. Entscheidend ist, dass sie sich an einer Komponente befindet, die drehfest an den Rotor angeschlossen ist, so dass die auf die Abgleitkante vom Schlauchsegment ausgeübte Kraft den Rotor in Drehbewegung versetzen kann. Vorteilhafterweise ist die Abgleitkante entweder Bestandteil eines Rotorkörpers, oder einer mit dem Rotor drehfest verbundenen Schwinge oder einer Rotorabdeckung mit einem die Quetschelemente übergreifenden Deckplatte.

Je tiefer die Abgleitkante liegt, d.h. je näher die Abgleitkante dem Pumpenbett liegt, desto früher kommt das Schlauchsegment beim Ausfädeln mit ihr in Kontakt. Deshalb ist es von Vorteil, wenn sich die Deckplatte der Rotorabdeckung zur Ausbildung der Abgleitkante in Umfangsrichtung entgegen der Drehrichtung verdickt.

Eine besonders einfach herzustellende Anordnung ergibt sich dann, wenn die Deckplatte zur Ausbildung der Abgleitkante einen nach unten abgewinkelten Randsteg aufweist.

Vorteile hinsichtlich der Montage der Quetschelemente ergeben sich, wenn der Randsteg an seinem radial äußersten Ende in einen Umfangskantenabschnitt übergeht, dessen Höhe sich zum Quetschelement hin allmählich verringert.

Vorzugsweise wird die Abgleitkante von einem Werkstoff gebildet und/oder beschichtet, der abriebminimierende Gleiteigenschaften hat.

Nachstehend werden Ausführungsbeispiele der Erfindung anhand schematischer Zeichnungen näher erläutert. Es zeigen:
Figur 1 eine perspektivische Darstellung einer beispielhaften Ausführungsform einer Peristaltikpumpe, wie sie in Verbindung mit einer Vorrichtung zur extrakorporalen Blutbehandlung Verwendung finden kann;
Figur 2 eine perspektivische Ansicht eines in der Peristaltikpumpe gemäß Figur 1 verwendeten Rotors;
Figur 3 eine schematische Ansicht der Peristaltikpumpe beim Ausfädeln eines Schlauchsegments aus dem Pumpenbett;
Figuren 4 und 5 Draufsichten der Peristaltikpumpe in verschiedenen Drehstellungen des Rotors, in denen ein einquetschsicheres Ausfädeln des Schlauchsegments ermöglicht ist;
Figur 6 eine schematische Ansicht einer bekannten gattungsbildenden Peristaltikpumpe zur Verdeutlichung des Drehlagefensters für ein sicheres Ausfädeln des Schlauchsegments;
Figur 7 eine der Figur 6 entsprechende Ansicht einer neuerungsgemäßen Ausführungsform der Peristaltikpumpe;
Figur 8 eine perspektivische Ansicht eines Rotors einer bekannten Peristaltikpumpe;
Figuren 9 und 10 Draufsichten einer bekannten Peristaltikpumpe in verschiedenen Drehstellungen des Rotors, in denen ein Ausfädeln des Schlauchsegments empfohlen wird; und
Figur 11 eine schematische Ansicht der bekannten Peristaltikpumpe mit einer für das Ausfädeln ungünstigen Drehposition des Rotors.

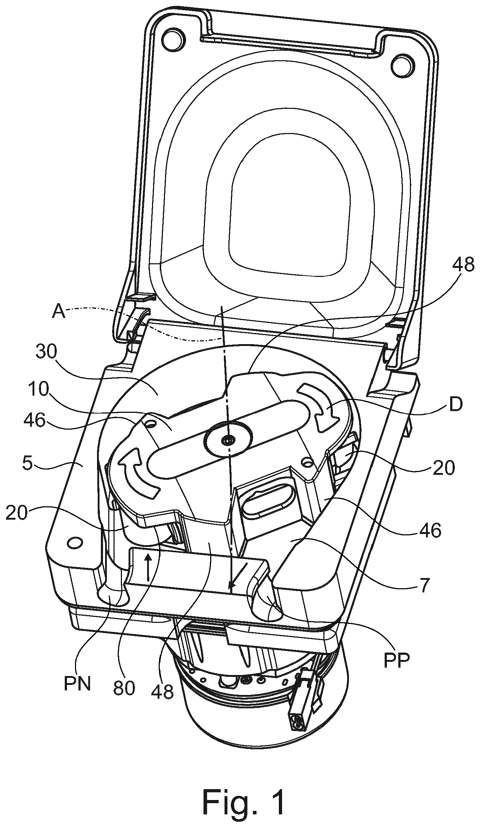

**In** den Figuren 1 bis 6 ist eine Peristaltikpumpe gezeigt, wie sie beispielsweise in Dialysemaschinen eingesetzt wird. Die Peristaltikpumpe hat dort die Aufgabe, ein definiertes Volumen eines Mediums, wie zum Beispiel Blut oder Dialysefluid, durch Verformen und Abklemmen der elastisch verformbaren Fluidleitung zu fördern. Die Peristaltikpumpe zur Förderung von Blut fördert in der Regel von einer negativen Druckseite PN (Niederdruckseite) auf eine positive Druckseite PP (Hochdruckseite).

Wie in Figur 1 dargestellt, hat die Peristaltikpumpe ein Pumpengehäuse 5, in dem ein um eine Rotorachse A rotierbarer Rotor 10 mit zumindest zwei in Umfangsrichtung zueinander beispielsweise diametral versetzten Quetschelementen 20 , hier Druckrollen, aufgenommen ist und welches eine sich bogenförmig um die Rotorachse A erstreckende und radial von dem Rotor 10 beabstandete Stützfläche 30 aufweist, die zum radialen Abstützen eines radial zwischen den Rotor 10 und die Stützfläche 30 einbringbaren, in Figur 1 nicht gezeigten Schlauchsegments eingerichtet ist. Die Quetschelemente 20 sind beispielsweise abgefedert auf einer nicht näher bezeichneten Schwinge gelagert, die ihrerseits schwenkbeweglich am Rotor 10 befestigt ist.

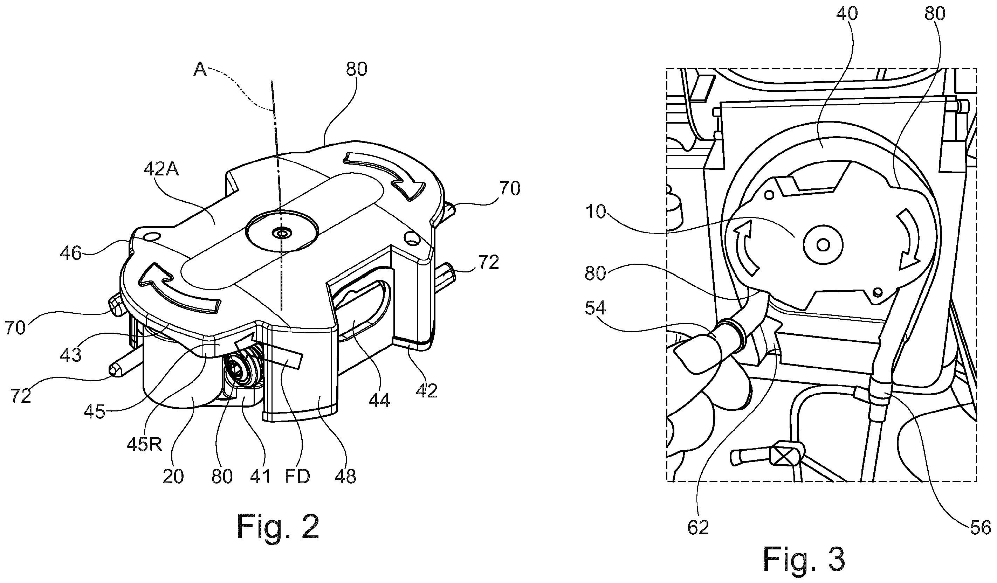

Das in der Peristaltikpumpe eingesetzte Schlauchsegment wird nachfolgend als Pumpensegment bezeichnet. Bodenseitig stützt sich das in Figur 1 nicht gezeigte Schlauchsegment nicht im mit dem Bezugszeichen 7 versehenen Pumpenbett ab, sondern wird durch zwei in die Schwinge integrierte Führungsstifte 70, 72 (siehe Figur 2) in mittiger Position zu den Quetschelementen 20 gehalten. Die Stützfläche 30 ist in der Regel von einer Zylinderfläche gebildet, sie kann jedoch auch muldenförmig vertieft sein. Die Drehrichtung des Rotors 10 beim Fördern von Fluid ist mit dem Pfeil D bezeichnet.

Figur 2 zeigt Einzelheiten des Rotors 10. Er hat einen nicht näher dargestellten Rotor-Grundkörper 42 und eine Rotorabdeckung 42A. Der Rotor 10 ist durch eine seitlich bedienbare Taste 44 von einer nicht näher gezeigten Antriebswelle abnehmbar. Die Quetschelemente 20 sind drehbar auf einer gelenkig am Rotor-Grundkörper 42 federnd abgestützten Schwinge 41 gelagert.

Die sich mit dem Rotor 10 mitdrehende Rotorabdeckung 42A trägt im Bereich der Quetschelemente 20 Führungsflächen 46, 48, von denen die eine Führungsfläche 46 dem Quetschelement 20 vorläuft und die andere Führungsfläche 48 dem Quetschelement 20 nachläuft. Die Führungsflächen 46, 48 sind demnach jeweils zu beiden Seiten des Quetschelements 20 in Umfangsrichtung benachbart derart angeordnet und gestaltet, dass sie - wie aus der Figuren 3 erkennbar - mit der Stützfläche 30 jeweils einen kreissegmentartigen Führungskanal FK1 und FK2 ausbilden, in dem das in das Pumpenbett 7 eingelegte Schlauchsegment 40, das auch als Pumpensegment bezeichnet wird, mit vorgegebener Spielpassung radial gefangen ist. Figur 3 lässt erkennen, dass das Pumpensegment 40 im Betrieb der Peristaltikpumpe am Pumpengehäuse 5 mittels zweier Passkörper 54, 56 fixiert sind, die an Anschlüssen 62, 64 des Pumpengehäuses 5 verschiebesicher fixierbar sind.

Wie der Darstellung gemäß Figur 2 entnommen werden kann, hat die Rotorabdeckung 42, also ein an den Rotor 10 drehfest angeschlossenes Teil, eine dem jeweiligen Quetschelement 20 entgegen der Drehrichtung D des Rotors 10 benachbart angeordnete, sich zur Stützfläche hin erstreckende Abgleitkante 80, die - wie der Figur 3 entnommen werden kann - vom eingelegten Schlauchsegment, also vom Pumpensegment 40 untergriffen ist.

Wenn das Pumpensegment 40 ausgefädelt werden soll, wird - wie in Figur 3 dargestellt - der an der Niederdruckseite PN liegende Passkörper 54 vom Pumpengehäuse 5 gelöst und vom Pumpenbett 7 nach oben gezogen. Der Rotor 10 soll dabei die in Figur 3 gezeigte Drehlage einnehmen. Weil das Pumpensegment 40 die Abgleitkante 80 untergreift, berührt das Pumpensegment 40 - wenn es wie in Figur 3 dargestellt nach oben gezogen wird - die Abgleitkante 80 in einem nach oben gebogenen Zustand, so dass die Kontaktkraft eine in Drehrichtung D des Rotors 10 gerichtete Kraftkomponente hat, die in Figur 2 mit dem Pfeil FD eingetragen ist. Wenn also das Pumpensegment 40 ausgefädelt werden soll, genügt eine kleine von Hand aufgebrachte Zugkraft, um den Rotor 10 in Richtung des Pfeils D zu drehen und das Pumpensegment 40 sukzessive freizugeben, so dass es aus dem Pumpenbett herausgezogen werden kann. Die auf das Pumpensegment 40 einwirkenden Zug- und Kontaktkräfte bleiben auf diese Weise relativ begrenzt, was der Haltbarkeit des Schlauchsegments zugutekommt.

Um das Pumpensegment 40 zusätzlich möglichst wenig zu beanspruchen, ist die Abgleitkante 80 von einem Werkstoff gebildet und/oder beschichtet, der abriebminimierende Gleiteigenschaften hat

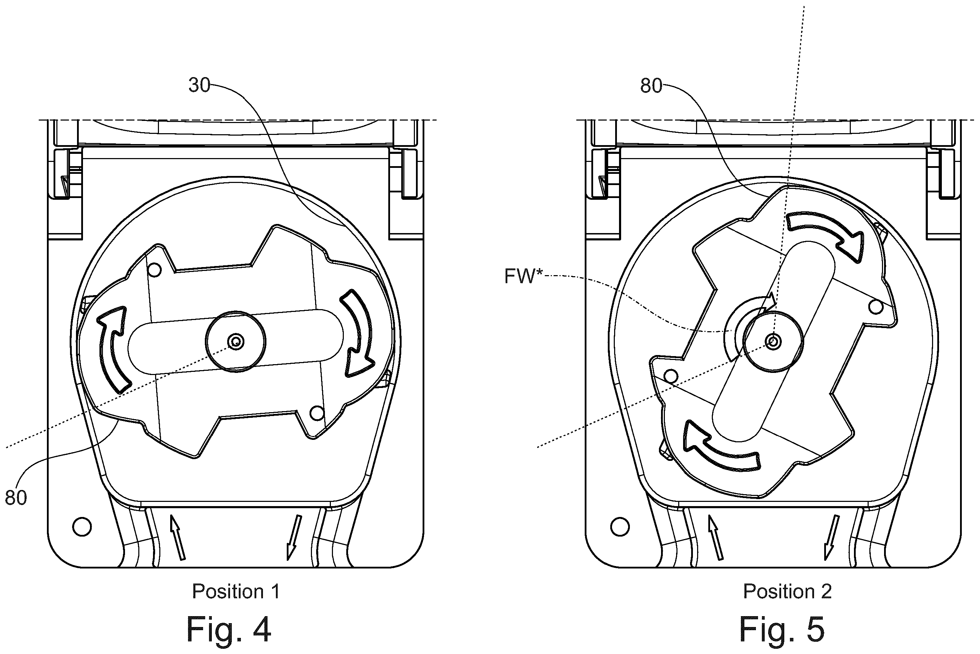

Die Abgleitkante 80 befindet sich im gezeigten Ausführungsbeispiel zwischen der Andruckrolle, also dem Quetschelement 20 und der diesem in Drehrichtung nachlaufenden Führungsfläche 48, also in unmittelbarer Nachbarschaft zum Quetschelement 20. Diese Anordnung führt dazu, dass der Bereich, in dem sich der Rotor 10 beim manuellen Ausfädeln befinden muss, um das Einklemmen des Pumpensegments 40 zu vermeiden, wesentlich größer als bei bekannten Peristaltikpumpen wird. Der Rotor 10 muss sich in einer Position, zwischen der in Figur 4 gezeigten Position 1 und der in Figur 5 gezeigten Position 2 befinden, die durch den Drehwinkel FW* voneinander getrennt sind, der in der Größenordnung von 120 bis 130° liegt. Das manuelle Ausfädeln durch die Bedienperson wird dadurch erheblich einfacher, weil sie nicht mehr verstärkt auf die Position des Rotors 10 achten muss.

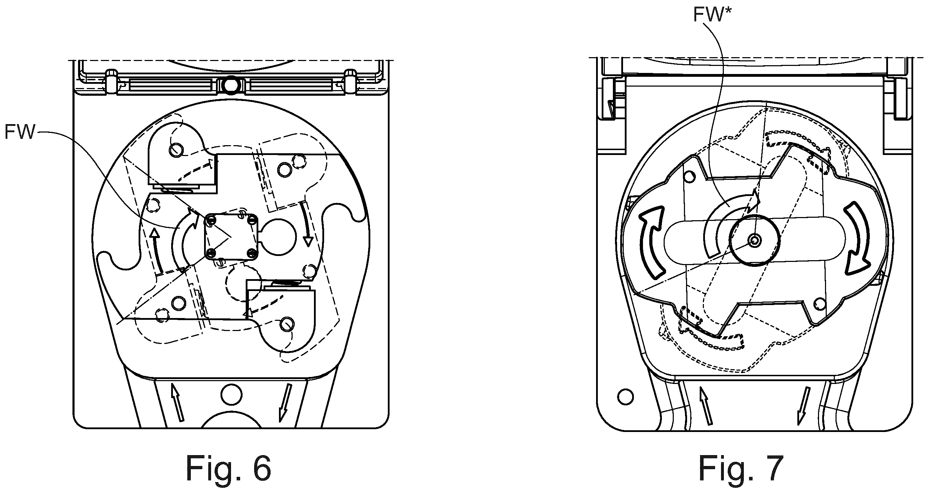

In den Figuren 6 und 7 sind die Drehpositionen, zwischen denen sich der Rotor 310 beim Stand der Technik und der Rotor 10 bei der anmeldungsgemäßen Ausgestaltung zum leichten Ausfädeln des Pumpensegments 40 bzw. 340 befinden muss, gegenübergestellt. In Figur 6 ist der bekannte Rotor 310 mit beiden Grenz-Drehpositionen gezeigt, in Figur 7 der anmeldungsgemäße Rotor 10. In einer ersten Drehposition ist der Rotor mit durchgezogenen Linien dargestellt, in der zweiten Grenz-Drehlage mit gestrichelten Linien. Man erkennt, dass sich das erlaubte Winkelfenster FW mit der anmeldungsgemäßen Positionierung und Ausgestaltung der Abgleitkante 80 erheblich auf den Wert FW* erweitern lässt.

Die Funktion der Abgleitkante 80 wird immer dann erfüllt, wenn sie an einer Komponente der Peristaltikpumpe ausgebildet oder angebracht ist, die drehfest mit dem Rotor 10 verbunden ist. Im gezeigten Ausführungsbeispiel ist diese Komponente von der Rotorabdeckung 42 gebildet, die eine die Quetschelemente 20 übergreifende Deckplatte 43 hat. Aus der Figur 2 ist ersichtlich, dass sich die Deckplatte 43 zur Ausbildung der Abgleitkante 80 in Umfangsrichtung entgegen der Drehrichtung D verdickt, wodurch die Abgleitkante 80 näher in Richtung auf das Pumpenbett 7 zu verlagert wird. Diese Verdickung kann beispielsweise dadurch realisiert werden, dass die Deckplatte 43 - wie in Figur 2 am besten dargestellt - zur Ausbildung der Abgleitkante 80 einen abgewinkelten Randsteg 45 aufweist. An seinem radial äußersten Ende 45R geht dabei der Randsteg 45 in einen Umfangskantenabschnitt über, dessen Höhe sich zum Quetschelement 20 hin allmählich verringert.

Selbstverständlich sind Abweichungen vom beschriebenen Ausführungsbeispiel möglich, ohne den Grundgedanken der Erfindung zu verlassen.

So kann die Abgleitkante 80 auch Bestandteil eines Rotorkörpers oder einer an den Rotor 10 angebunden, die Quetschelemente tragenden Schwinge sein.

Bei der vorstehend beschriebenen Peristaltikpumpe sind zwei Quetschelemente vorgesehen, die in einer relativen Position von 180° starr bzw. in einem festen Winkel von 180° zueinander angeordnet sind. Es kann jedoch auch vorgesehen sein, dass mehr als zwei Quetschelemente vorhanden sind und dass die Quetschelemente in Rotationsrichtung relativ zueinander winkelpositionierbar ausgebildet sind. Hierzu können die Quetschelemente auf Rotorarmen angeordnet sein, die gegenüber dem Rotor in Umfangsrichtung verschwenkbar ausgebildet sein können.

Die Quetschelemente müssen nicht als Quetschrollen oder Andruckrollen, die in vorteilhafter Weise materialschonend an der Fluidleitung abrollen, ausgebildet sein. Es können auch Gleitschuhe, die sich gleitend über das Schlauchsegment bewegen, vorgesehen sein.

Die Erfindung schafft somit Peristaltikpumpe, insbesondere zum Fördern von Fluid in einer Vorrichtung zur extrakorporalen Blutbehandlung, mit einem Pumpengehäuse, in dem ein um eine Rotorachse rotierbarer Rotor mit zumindest zwei in Umfangsrichtung zueinander versetzten Quetschelementen aufgenommen ist und welches eine sich bogenförmig um die Rotorachse erstreckende und radial von dem Rotor beabstandete Stützfläche aufweist, die zum Abstützen eines radial zwischen den Rotor und die Stützfläche einbringbaren Schlauchsegments eingerichtet ist. Zur Vereinfachung des manuellen Ausfädelns des Schlauchsegments, hat die Peristaltikpumpe eine dem jeweiligen Quetschelement entgegen der Drehrichtung des Rotors benachbart angeordnete, sich zur Stützfläche hin erstreckende Abgleitkante, die an den Rotor drehfest angeschlossen und vom eingelegten Schlauchsegment untergreifbar ist.

### Bezugszeichenliste

- A: Rotorachse
- WZF: Zentriwinkel der Führungsfläche
- D: Drehrichtung
- PN: Niederdruckseite
- PP: Hochdruckseite
- FW, FW*: Winkelfenster
- FD: Kraftkomponente in Drehrichtung

- 5: Pumpengehäuse
- 7: Pumpenbett
- 10: Rotor
- 20: Quetschelement
- 30: Stützfläche
- 40: Pumpensegment
- 42: Rotor-Grundkörper
- 42A: Rotorabdeckung
- 43: Deckplatte
- 44: Taste
- 45: Randsteg
- 46: Führungsfläche
- 48: Führungsfläche
- 54, 56: Passkörper
- 62, 64: Anschlüsse
- 70: oberer Führungsstift
- 72: unterer Führungsstift

- 80: Abgleitkante

- 310: Rotor
- 320: Quetschelemente
- 330: Stützfläche
- 340: Pumpensegment
- 350: Führungsfläche
- 360: Führungsvorsprünge
- 370: Kanten

## Patentansprüche

1. Peristaltikpumpe, insbesondere zum Fördern von Fluid in einer Vorrichtung zur extrakorporalen Blutbehandlung, mit
einem Pumpengehäuse (5), in dem ein um eine Rotorachse (A) rotierbarer Rotor (10) mit zumindest zwei in Umfangsrichtung zueinander versetzten Quetschelementen (20) aufgenommen ist und welches eine sich bogenförmig um die Rotorachse (A) erstreckende und radial von dem Rotor (10) beabstandete Stützfläche (30) aufweist, die zum Abstützen eines radial zwischen den Rotor (10) und die Stützfläche (30) einbringbaren Schlauchsegments (40) eingerichtet ist, **gekennzeichnet durch**
eine dem jeweiligen Quetschelement (20) entgegen der Drehrichtung (D) des Rotors (10) benachbart angeordnete, sich zur Stützfläche (30) hin erstreckende Abgleitkante (80), die an den Rotor (10) drehfest angeschlossen und vom eingelegten Schlauchsegment (40) untergreifbar ist.

2. Peristaltikpumpe nach Anspruch 1, **dadurch gekennzeichnet, dass** die Abgleitkante (80) Bestandteil eines Rotorkörpers (42), einer mit dem Rotor (10) drehfest verbundenen Schwinge oder einer Rotorabdeckung (42A) mit einem die Quetschelemente (20) übergreifenden Deckplatte (43) ist.

3. Peristaltikpumpe nach Anspruch 2, **dadurch gekennzeichnet, dass** sich die Deckplatte (43) zur Ausbildung der Abgleitkante (80) in Umfangsrichtung entgegen der Drehrichtung (D) verdickt.

4. Peristaltikpumpe nach Anspruch 3, **dadurch gekennzeichnet, dass** die Deckplatte (43) zur Ausbildung der Abgleitkante (80) einen abgewinkelten Randsteg (45) aufweist.

5. Peristaltikpumpe nach Anspruch 4, **dadurch gekennzeichnet, dass** der Randsteg (45) an seinem radial äußersten Ende (45R) in einen Umfangskantenabschnitt übergeht, dessen Höhe sich zum Quetschelement (20) hin allmählich verringert.

6. Peristaltikpumpe nach einem der Ansprüche 1 bis 5, **dadurch gekennzeichnet, dass** die Abgleitkante (80) von einem Werkstoff gebildet und/oder beschichtet ist, der abriebminimierende Gleiteigenschaften hat.

## Claims

1. A peristaltic pump, in particular for conveying fluid in a device for extracorporeal blood treatment, the peristaltic pump comprising:
a pump housing (5) in which a rotor (10) rotatable about a rotor axis (A) with at least two squeeze elements (20) offset in a circumferential direction to each other is accommodated and which has a support surface (30) extending arc-shaped about the rotor axis (A) and spaced radially from the rotor (10), the support surface being configured to support a tube segment (40) that can be introduced radially between the rotor (10) and the support surface (30); **characterized by**
a sliding edge (80) which is arranged adjacent to the respective squeeze element (20) against a rotational direction (D) of the rotor (10), extends toward the support surface (30) and is connected to the rotor in a rotationally fixed manner and is configured to be gripped from below by the tube segment (40).

2. The peristaltic pump according to claim 1, **characterized in that** the sliding edge (80) is a component of a rotor body (42), of a swing arm connected to the rotor (10) in a rotationally fixed manner or of a rotor cover (42A) with a cover plate (43) overlapping the squeeze elements (20).

3. The peristaltic pump according to claim 2, **characterized in that** the cover plate (43) thickens in the circumferential direction against the rotational direction to form the sliding edge (80).

4. The peristaltic pump according to claim 3, **characterized in that** the cover plate (43) has an angled border bar (45) to form the sliding edge (80).

5. The peristaltic pump according to claim 4, **characterized in that** the border bar (45) at its radially outermost end (45R) merges into a circumferential edge portion, the height of which gradually decreases toward the squeeze element (20).

6. The peristaltic pump according to any of claims 1 to 5, **characterized in that** the sliding edge (80) is formed and/or coated by a material that has abrasion-minimizing sliding properties.

## Revendications

1. Pompe péristaltique, en particulier pour transporter du fluide dans un dispositif de traitement sanguin extracorporel, avec
un boîtier de pompe (5), dans lequel est logé un rotor (10) pouvant tourner autour d'un axe de rotor (A) avec au moins deux éléments de pressage (20) décalés l'un par rapport à l'autre en direction circonférentielle et qui présente une surface d'appui (30) s'étendant en forme d'arc autour de l'axe de rotor (A) et espacée radialement du rotor (10), surface d'appui qui est configurée pour soutenir un segment de tuyau (40) pouvant être introduit radialement entre le rotor (10) et la surface d'appui (30), **caractérisée en ce que**
une arête de glissement (80) s'étendant vers la surface d'appui (30), agencée de manière contigüe à l'élément de pressage (20) respectif dans le sens inverse au sens de rotation (D) du rotor (10), arête qui est raccordée sans pouvoir tourner au rotor (10) et peut être saisie par en dessous par le segment de tuyau (40) inséré.

2. Pompe péristaltique sur la revendication 1, **caractérisée en ce que** l'arête de glissement (80) fait partie d'un corps de rotor (42), d'un bras oscillant relié sans pouvoir tourner au rotor (10) ou d'un couvercle de rotor (42A) avec une plaque de recouvrement (43) recouvrant les éléments de pressage (20).

3. Pompe péristaltique sur la revendication 2, **caractérisée en ce que** la plaque de recouvrement (43) s'épaissit pour former l'arête de glissement (80) dans le sens circonférentiel dans le sens inverse au sens de rotation (D).

4. Pompe péristaltique sur la revendication 3, **caractérisée en ce que** la plaque de recouvrement (43) présente une nervure de bord (45) coudée pour former l'arête de glissement (80).

5. Pompe péristaltique sur la revendication 4, **caractérisée en ce que** la nervure de bord (45) passe à son extrémité radialement la plus extérieure (45R) en une section d'arête circonférentielle dont la hauteur diminue progressivement vers l'élément de pressage (20).

6. Pompe péristaltique selon l'une quelconque des revendications 1 à 5, **caractérisée en ce que** l'arête de glissement (80) est formée et/ou revêtue d'un matériau qui présente des propriétés de glissement minimisant l'abrasion.
